(19) Europäisches Patentamt / European Patent Office / Office européen des brevets

(11) **EP 3 641 865 B1**

(12) **EUROPÄISCHE PATENTSCHRIFT**

(45) Veröffentlichungstag und Bekanntmachung des Hinweises auf die Patenterteilung:
**07.07.2021   Patentblatt 2021/27**

(21) Anmeldenummer: **18829248.6**

(22) Anmeldetag: **12.12.2018**

(51) Int Cl.:
*A61M 16/00* (2006.01)          *A61M 16/08* (2006.01)
*A61M 16/22* (2006.01)          *A61M 16/20* (2006.01)
*A61M 16/10* (2006.01)          *A61B 5/08* (2006.01)

(86) Internationale Anmeldenummer:
**PCT/EP2018/084444**

(87) Internationale Veröffentlichungsnummer:
**WO 2019/115571 (20.06.2019 Gazette 2019/25)**

(54) **VERFAHREN, COMPUTERPROGRAMM, VORRICHTUNG UND BEATMUNGSSYSTEM ZUR DETEKTION EINES LECKS IN EINEM PATIENTENGASMODUL**

METHOD, COMPUTER PROGRAM, DEVICE, AND VENTILATION SYSTEM FOR DETECTING A LEAK IN A PATIENT GAS MODULE

PROCÉDÉ, PROGRAMME INFORMATIQUE, DISPOSITIF ET SYSTÈME DE VENTILATION PERMETTANT LA DÉTECTION D'UNE FUITE DANS UN MODULE DE GAZ DE PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.12.2017   DE 102017011625**

(43) Veröffentlichungstag der Anmeldung:
**29.04.2020   Patentblatt 2020/18**

(73) Patentinhaber: **Drägerwerk AG & Co. KGaA
23558 Lübeck (DE)**

(72) Erfinder:
• **PETER, Gerd
  23562 Lübeck (DE)**
• **SCHMID, Uwe
  23564 Lübeck (DE)**
• **OSTERLOH, Christoph
  23628 Klempau (DE)**

(56) Entgegenhaltungen:
EP-A1- 1 961 439          EP-A1- 1 974 763
WO-A1-2012/085753          WO-A1-2014/068000

**Beschreibung**

Ausführungsbeispiele beziehen sich auf ein Verfahren, ein Computerprogramm, eine Vorrichtung und ein Beatmungssystem zur Detektion eines Lecks in einem Patientengasmodul, insbesondere aber nicht ausschließlich, auf ein Konzept zur Leckdetektion basierend auf einem Vergleich von Konzentrationsverläufen verschiedener Gase in einem Atemgasgemisch in einem Patientengasmodul oder Beatmungssystem.

Aus dem Bereich der konventionellen Technik ist es bekannt Patienten künstlich zu beatmen. Dabei wird dem Patienten ein Beatmungsgas in einer Inspirationsphase zugeführt und in einer Expirationsphase wieder abgeführt. Dabei können diverse Parameter des Atemgases überwacht werden und es kommen beispielsweise sogenannte Patientengasmodule zur Gasanalyse zum Einsatz. Z.B. kann ein Teil des Gases jeweils abgesaugt werden, um es einer Gasanalyse in einem Patientenmodul zuzuführen.

Bei der absaugenden Patientengasmessung, die über eine Probengasleitung einen kontinuierlichen Gasstrom vom Patienten absaugt, verursachen Leckagen auf der Absaugstrecke (z.B. via Probengasleitung, Wasserfalle und Gasmonitor-interne Verschlauchung bis zur Gasmesszelle) Verdünnungseffekte durch eindringende Umgebungsluft, die zu Gaskonzentrationsmessfehlern führen. Dies ist besonders problematisch bei Anästhesiearbeitsplätzen, die mit Regelkreisen ausgestattet sind: Hier werden die (potentiell mit o. g. Fehlern behafteten) Konzentrationsmesswerte zur Regelung für die Gasdosierung verwendet. Treten hier Messfehler auf, die nicht entdeckt werden, so kommt es zu Fehldosierungen die u. U. sogar zu einem Patientenschaden führen können.

Die Druckschrift US 8,033,280 B2 beschreibt ein Verfahren zur Bestimmung einer Leckage in der Probengasleitung, indem die Konzentrationen von mindestens zwei Gasen gemessen und gleichzeitige Änderungen dieser Konzentrationen in Richtung bekannter Umgebungsgaskonzentrationen identifiziert werden. Diese Änderungen werden dann als Leckage interpretiert. Die zwei Gase sind idealerweise Kohlendioxid CO2 und Sauerstoff 02. Diese Überwachung misst relativ und ermittelt die Konzentrationsänderung zwischen aktueller und Vorgänger-Atemphase. Wenn die Leckage abrupt zwischen zwei Atemphasen mit genügend großem Verdünnungseffekt auftritt kann diese so detektiert werden. Langsam entstehende Leckagen können nur schwierig erkannt werden.

Das Dokument WO 2014/068000 A1 beschäftigt sich mit Leckage-Detektion durch eine Druckmessung in einem Patientenmonitor und Vergleich mit einer Druckmessung in einem Kreissystem. Dabei kann es vorkommen, dass Änderungen der Widerstände der Probengasleitung/Wasserfalle den Effekt der Atemwegsdruckänderungen in dem Patientenmonitor überdecken. Bei laufender Pumpe können die Druckänderungen stark gedämpft werden und führen ggf. nur für genügend hohe Atemwegsdruck-Einstellwerte zu einer aussagekräftigen Überwachung. In der Druckschrift WO 2004/076944 A2 wird dieser Ansatz mit zwei verteilten Sensoren verfolgt.

Es besteht daher ein Bedarf ein verbessertes Konzept zur Detektion eines Lecks in einem Beatmungssystem zur Beatmung eines Patienten zu schaffen. Diesem Bedarf werden Ausführungsbeispiele eines Verfahrens, eines Computerprogramms, einer Vorrichtung und einem Beatmungssystem gemäß den anhängigen unabhängigen Ansprüchen gerecht.

Ausführungsbeispiele basieren auf der Erkenntnis, dass mehrere Gaskonzentrationen in einem Gasgemisch einer Beatmungsvorrichtung gemessen werden können. Die zeitlichen Verläufe der Gaskonzentrationen werden im Falle eines Lecks unterschiedlich beeinflusst, sodass zeitliche Konzentrationsverläufe die Grundlage einer Leckagedetektion bilden können. Darüber hinaus basieren Ausführungsbeispiele auf der Erkenntnis, dass der Verlauf einer Kohlendioxidkonzentration in einem Atemgasgemisch eines Patienten eine Referenz zum Vergleich mit einem Konzentrationsverlauf eines anderen Gases in dem Atemgasgemisch des Patienten bilden kann.

Ausführungsbeispiele schaffen daher ein Verfahren zur Detektion eines Lecks in einem Patientengasmodul, das einen kontinuierlichen Probengasstrom von einem beatmeten Patienten absaugt und analysiert, beispielsweise auch in einem Beatmungssystem zur Beatmung eines Patienten mit einem Beatmungsgasgemisch. Beispielsweise wird hier durch die Leckage von z. B. 40 ml/min (bei einem abgesaugtem Probengasfluss von 200 ml/min) Messfehler von bis zu 20% in der Gaskonzentrationsmessung verursacht, während sie für die Beatmung von einem Patienten mit einem Atemminutenvolumen von ca. 8 l völlig irrelevant ist. Das Verfahren umfasst ein Bestimmen eines ersten zeitlichen Verlaufs einer Kohlendioxidkonzentration in einem Atemgasgemisch des Patienten und ein Bestimmen eines zweiten zeitlichen Verlaufs einer Konzentration eines anderen, sich von Kohlendioxid unterscheidenden Gases in dem Atemgasgemisch. Das Verfahren umfasst ferner ein Bestimmen eines statistischen Ähnlichkeitsmaßes zwischen dem ersten und dem zweiten zeitlichen Verlauf und ein Detektieren des Lecks basierend auf dem Ähnlichkeitsmaß. Ausführungsbeispiele können so eine zuverlässige Leckagedetektion bereitstellen. Dabei kann das andere Gas beispielsweise Sauerstoff, Lachgas oder ein Narkosegas sein.

Erfindungsgemäß entspricht das Ähnlichkeitsmaß einer Phasenverschiebung zwischen dem ersten und dem zweiten Verlauf. Ausführungsbeispiele können so eine robuste Detektion eines Lecks ermöglichen. Das Bestimmen des Ähnlichkeitsmaßes kann ein Bestimmen einer für ein Leck eigentümlichen Verformung in dem ersten oder dem zweiten Verlauf umfassen. In einigen Ausführungsbeispielen kann das Ähnlichkeitsmaß ein Maß für eine Symmetrie zwischen dem ersten und dem zweiten Verlauf sein. Das Ähnlichkeitsmaß kann eine Kovarianz zwischen dem ersten und dem zweiten zeitlichen Verlauf der Konzentrationen sein. Zumindest manche Ausführungsbeispiele können so eine statistische Auswertung nutzen, um ein Leck zu detektieren. In weiteren Ausführungsbeispielen kann das Ähnlichkeitsmaß eine Kovarianz zwischen einem ersten und einem zweiten zeitlichen Verlauf von Konzentrationsänderungen sein.

In manchen Ausführungsbeispielen kann ein Vergleichen des Ähnlichkeitsmaßes mit einem Schwellenwert und ein Detektieren des Lecks basierend auf dem Schwellenwertvergleich erfolgen. Ausführungsbeispiele können so eine aufwandsgünstige Signalverarbeitung ermöglichen. Darüber hinaus kann in einigen Ausführungsbeispielen ein Kalibrieren des Schwellenwertes basierend auf einer Referenzmessung an dem Beatmungssystem ohne Leck erfolgen. Die Zuverlässigkeit des Verfahrens kann so noch weiter gesteigert werden. In manchen Ausführungsbeispielen kann darüber hinaus ein Bestimmen einer Laufzeit des Atemgasgemischs von dem Patienten über eine Probengasleitung zu einem Patientengasmodul erfolgen. Die Laufzeit kann bei der Detektion eines Lecks mit berücksichtigt werden. Insofern können Ausführungsbeispiele ein Bestimmen einer Laufzeit von Konzentrationsänderungen in dem Atemgasgemisch und ein Bestimmen des Ähnlichkeitsmaßes basierend auf den Laufzeiten umfassen.

Das Bestimmen der Laufzeit kann auf Konzentrationsänderungen über der Zeit und/oder auf einer Bewertung von Konzentrationsänderungen vor und nach einem Beginn von Atemphasen basieren. In einigen weiteren Ausführungsbeispielen kann das Verfahren ferner ein Durchführen einer Druckmessung an dem Beatmungssystem umfassen, wenn ein Leck detektiert wurde. Die Druckmessung kann in Ausführungsbeispielen bei der Leckdetektion oder auch bei der einer Leckortung berücksichtigt werden.

Ein weiteres Ausführungsbeispiel ist ein Computerprogramm mit einem Programmcode zur Durchführung eines der hierin beschriebenen Verfahren, wenn der Programmcode auf einem Computer, einem Prozessor oder einer programmierbaren Hardwarekomponente ausgeführt wird. Weitere Ausführungsbeispiele sind eine Vorrichtung zum Durchführen eines der hierin beschriebenen Verfahren und ein Beatmungssystem für einen Patienten mit einer solchen Vorrichtung.

Weitere vorteilhafte Ausgestaltungen werden nachfolgend anhand der in den Zeichnungen dargestellten Ausführungsbeispiele, auf welche Ausführungsbeispiele generell jedoch nicht insgesamt beschränkt sind, näher beschrieben. Es zeigen:

Fig. 1 zeigt ein Ablaufdiagramm eines Ausführungsbeispiels eines Verfahrens zur Detektion eines Lecks in einem Beatmungssystem zur Beatmung eines Patienten;

Fig. 2 zeigt ein Beatmungssystem zur Beatmung eines Patienten in einem Ausführungsbeispiel;

Fig. 3 zeigt ein Kapnogramm in einem Ausführungsbeispiel;

Fig. 4 zeigt Konzentrationsverläufe von Kohlendioxid und Sauerstoff in einem Ausführungsbeispiel;

Fig. 5 zeigt einen Kohlendioxid-Konzentrationsverlauf und einen Kohlendioxid-Sauerstoff-Kovarianzverlauf in einem Ausführungsbeispiel;

Fig. 6 zeigt Konzentrationsverläufe und Druckverläufe in einem Patientengasmodul in einem Ausführungsbeispiel ohne Leckage;

Fig. 7 zeigt Konzentrationsverläufe und Druckverläufe in einem Patientengasmodul in einem Ausführungsbeispiel mit Leckage; und

Fig. 8 zeigt Konzentrationsverläufe und Druckverläufe in einem Patientengasmodul in einem weiteren Ausführungsbeispiel mit Leckage.

Verschiedene Ausführungsbeispiele werden nun ausführlicher unter Bezugnahme auf die beiliegenden Zeichnungen beschrieben, in denen einige Ausführungsbeispiele dargestellt sind.

Bei der nachfolgenden Beschreibung der beigefügten Figuren, die lediglich einige exemplarische Ausführungsbeispiele zeigen, können gleiche Bezugszeichen gleiche oder vergleichbare Komponenten bezeichnen. Ferner können zusammenfassende Bezugszeichen für Komponenten und Objekte verwendet werden, die mehrfach in einem Ausführungsbeispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt. Optionale Komponenten sind in den Figuren mit gestrichelten Linien oder Pfeilen dargestellt.

Obwohl Ausführungsbeispiele auf verschiedene Weise modifiziert und abgeändert werden können, sind Ausführungsbeispiele in den Figuren als Beispiele dargestellt und werden hierin ausführlich beschrieben. Es sei jedoch klargestellt, dass nicht beabsichtigt ist, Ausführungsbeispiele auf die jeweils offenbarten Formen zu beschränken, sondern dass Ausführungsbeispiele vielmehr sämtliche funktionale und/oder strukturelle Modifikationen, Äquivalente und Alternativen, die im Bereich der Erfindung liegen, abdecken sollen. Gleiche Bezugszeichen bezeichnen in der gesamten Figurenbeschreibung gleiche oder ähnliche Elemente.

Man beachte, dass ein Element, das als mit einem anderen Element "verbunden" oder "verkoppelt" bezeichnet wird, mit dem anderen Element direkt verbunden oder verkoppelt sein kann oder dass dazwischenliegende Elemente vorhanden sein können. Wenn ein Element dagegen als "direkt verbunden" oder "direkt verkoppelt" mit einem anderen Element bezeichnet wird, sind keine dazwischenliegenden Elemente vorhanden. Andere Begriffe, die verwendet werden, um die Beziehung zwischen Elementen zu beschreiben, sollten auf ähnliche Weise interpretiert werden (z.B., "zwischen" gegenüber "direkt dazwischen", "angrenzend" gegenüber "direkt angrenzend" usw.).

Die Terminologie, die hierin verwendet wird, dient nur der Beschreibung bestimmter Ausführungsbeispiele und soll die Ausführungsbeispiele nicht beschränken. Wie hierin verwendet, sollen die Singularformen "einer", "eine", "eines" und "der, die, das" auch die Pluralformen beinhalten, solange der Kontext nicht eindeutig etwas anderes angibt. Ferner sei klargestellt, dass die Ausdrücke wie z.B. "beinhaltet", "beinhaltend", "aufweist", "umfasst", "umfassend" und/oder "aufweisend", wie hierin verwendet, das Vorhandensein von genannten Merkmalen, ganzen Zahlen, Schritten, Arbeitsabläufen, Elementen und/oder Komponenten angeben, aber das Vorhandensein oder die Hinzufügung von einem bzw. einer oder mehreren Merkmalen, ganzen Zahlen, Schritten, Arbeitsabläufen, Elementen, Komponenten und/oder Gruppen davon nicht ausschließen.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

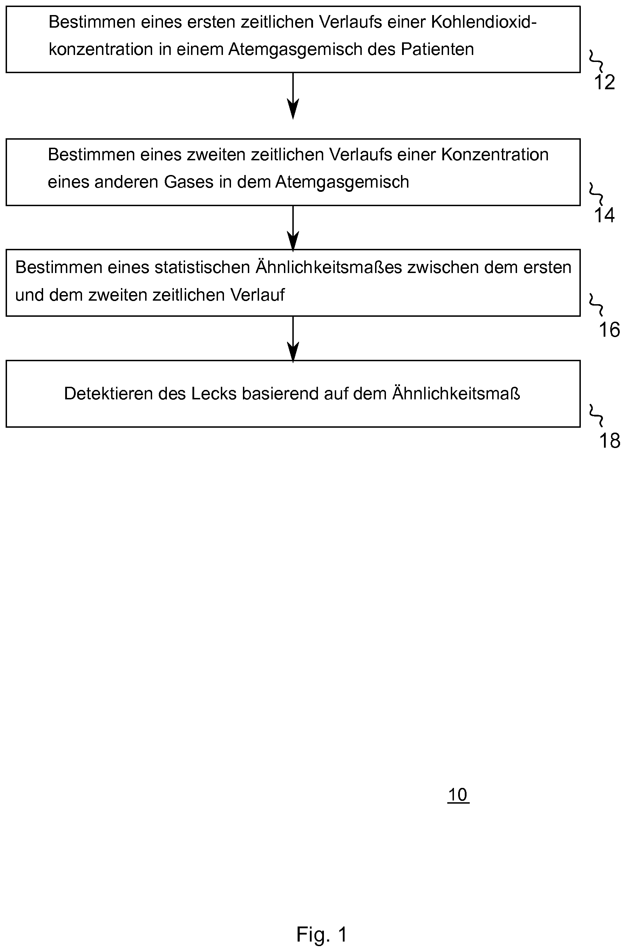

Fig. 1 zeigt ein Ablaufdiagramm eines Ausführungsbeispiels eines Verfahrens 10 zur Detektion eines Lecks in einem Patientengasmodul, das einen kontinuierlichen Probengasstrom von einem beatmeten Patienten absaugt und analysiert, beispielsweise in einem Beatmungssystem zur Beatmung eines Patienten. Das Verfahren 10 umfasst ein Bestimmen 12 eines ersten zeitlichen Verlaufs einer Kohlendioxidkonzentration in einem Atemgasgemisch des Patienten und ein Bestimmen 14 eines zweiten zeitlichen Verlaufs einer Konzentration eines anderen, sich von Kohlendioxid unterscheidenden Gases in dem Atemgasgemisch. Das Verfahren umfasst ferner ein Bestimmen 16 eines statistischen Ähnlichkeitsmaßes zwischen dem ersten und dem zweiten zeitlichen Verlauf und ein Detektieren 18 des Lecks basierend auf dem Ähnlichkeitsmaß.

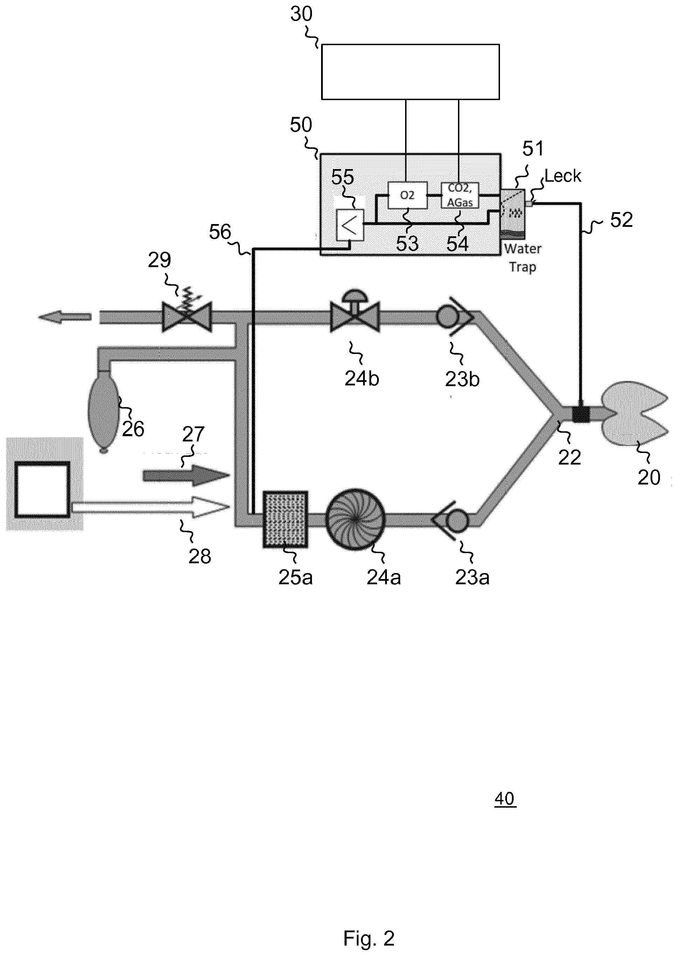

Fig. 2 zeigt ein Beatmungssystem 40 zur Beatmung eines Patienten 20 in einem Ausführungsbeispiel. Der Patient 20 ist über ein Mundstück und ein Verzweigungs- oder auch Y-Stück 22 an zwei Zweige zur Zufuhr (Einatmung, Inspiration) und Abfuhr (Ausatmung, Exspiration) des Atemgases angeschlossen. Im unteren Zweig, der für die Zufuhr vorgesehen ist, befindet sich ein Rückschlagventil 23a, das Gas in Richtung des Patienten 20 durchlässt und in Gegenrichtung sperrt. Ein Gebläse 24a sorgt für einen entsprechenden Volumenstrom in Richtung des Patienten 20. Dem Gebläse 24a vorgeschaltet ist ein Kohlendioxid-Absorber 25a, der Kohlendioxid aus dem zugeführten Atemgasgemisch absorbiert. Im oberen Zweig des Beatmungssystems 40, der für die Abfuhr des Atemgases vorgesehen ist, befindet sich ebenfalls ein Rückschlagventil 23b, das Atemgas in Richtung vom Patienten 20 weg durchlässt und in Richtung zum Patienten 20 hin sperrt. Das Rückschlagventil 23b wird gefolgt von einem PEEP-Ventil 24b (von englisch "positive end-expiratory pressure", positiver endexspiratorischer Druck). Das PEEP-Ventil 24b sorgt während der Inspirationsphase gemeinsam mit dem Gebläse für einen regelbaren positiven Druck in der Lunge des Patienten 20 und öffnet bzw. hält einen wählbaren Restdruck während der Exspirationsphase. Der obere und der untere Zweig des Beatmungssystems 40 können über ein T-Stück verbunden sein, das ferner an einen Atembeutel 26 angeschlossen ist, über den der Beatmungskreislauf angetrieben werden kann. Darüber hinaus kann Frischluft/Frischgas 27 und/oder dampfförmiges Anästhesiemittel 28 dem Kreislauf zugeführt werden.

Wie die Fig. 2 weiter zeigt, ist das Beatmungssystem 40 über ein weiteres Ventil 29 mit weiteren Komponenten verbindbar, beispielsweise zur Reinigung des Atemgases. Bei dem Ventil 29 kann es sich um ein APL-Ventil (von englisch "Adjustable-Pressure-Limiting", einstellbares Überdruckventil) handeln, das einerseits eine Drucklimitierung bei volumenkontrollierter Beatmung und andererseits die Spontanatmung des Patienten ermöglichen kann. Wenn der eingestellte Maximaldruck erreicht wird, öffnet das Ventil 29.

Die Fig. 2 illustriert ferner ein Ausführungsbeispiel einer Vorrichtung 30 zum Durchführen eines der hierin beschriebenen Verfahren 10, die mit einem Patientengasmodul 50 gekoppelt ist. Das Patientengasmodul 50 ist über eine Wasserfalle 51, zur Abscheidung von Kondenswasser und Verunreinigungen, und einen Schlauch 52 mit dem Beatmungssystem 40 gekoppelt. An diesem Schlauch bzw. an dessen Anschluss an die Wasserfalle 51 kann beispielsweise ein Leck auftreten. In dem Patientengasmodul 50 werden im vorliegenden Ausführungsbeispiel Gaskonzentrationen bestimmt 53, 54 und eine Pumpe 55 fördert das Gas über einen Rückführschlauch 56 zurück in das System. Gemäß der Fig. 1 wird vorliegend eine Kohlendioxidkonzentration (CO2) bestimmt und zumindest eine Konzentration eines anderen Gases. Das andere Gas kann beispielsweise Sauerstoff (02), Lachgas (N2O) oder ein Narkosegas (Agas) sein.

Die Konzentrationen werden dann der Vorrichtung 30 bereitgestellt und gemäß dem Verfahren 10 der Fig.1 verarbeitet. Ein weiteres Ausführungsbeispiel ist ein Computerprogramm mit einem Programmcode zur Durchführung eines der hierin beschriebenen Verfahren, wenn der Programmcode auf einem Computer, einem Prozessor oder einer programmierbaren Hardwarekomponente ausgeführt wird. Die Vorrichtung 30 kann insofern als Computer, Prozessor oder programmierbare Hardwarekomponente implementiert sein. In Ausführungsbeispielen kann die Vorrichtung einem beliebigen Controller oder Prozessor oder einer programmierbaren Hardwarekomponente entsprechen. Das Verfahren 10 kann als Software realisiert sein, die für eine entsprechende Hardwarekomponente programmiert ist. Insofern kann die Vorrichtung als programmierbare Hardware mit entsprechend angepasster Software implementiert sein. Dabei können beliebige Prozessoren, wie Digitale Signal-Prozessoren (DSPs) zum Einsatz kommen. Ausführungsbeispiele sind dabei nicht auf einen bestimmten Typ von Prozessor eingeschränkt. Es sind beliebige Prozessoren oder auch mehrere Prozessoren zur Implementierung der Vorrichtung 30 denkbar.

Die Vorrichtung 30 kann demnach zur Verarbeitung von Abtastwerten der einzelnen Konzentrationen ausgebildet sein. In Ausführungsbeispielen werden die Konzentrationen mit entsprechend abgetastet, wobei die Abtastrate entsprechend der Vermeidung von Aliasing gewählt wird. Beispielsweise werden die Konzentrationen alle 20ms (Abtastrate von 50Hz) bestimmt. In einem Ausführungsbeispiel finden die schnellsten zu erwartenden Änderungen über einen Zeitraum von 100ms (300ms bei CO2) statt, sodass bei der gewählten Abtastrate kein Aliasing zu erwarten ist. Darüber hinaus können verschiedene Sensoren für die verschiedenen Gase zum Einsatz kommen, die vorzugsweise in unmittelbarer Nähe zueinander angeordnet sind, um zeitliche Verzögerungen (aufgrund der Ausbreitungsgeschwindigkeit) bei den Messungen der Konzentrationen gering zu halten und zeitlich synchronisierte Messwerte zu erhalten. In anderen Ausführungsbeispielen können beabstandete Sensoren verwendet werden, wobei die Abstände basierend auf der Ausbreitungsgeschwindigkeit des Gases bei den Messungen berücksichtigt werden können.

In dem in der Fig. 2 gezeigten Ausführungsbeispiel kann beispielsweise eine kontinuierliche Überwachung der absaugenden Messung bei maximaler Verfügbarkeit der Gasmessung sichergestellt werden. Generell kann in Ausführungsbeispielen das Verfahren 10 daher auch ein Absaugen des Atemgasgemischs über eine Probegasleitung 52 umfassen. In manchen Ausführungsbeispielen können auffällige Zeitverläufe der CO2-Konzentration Grundlage für die Erkennung einer Leckage sein.

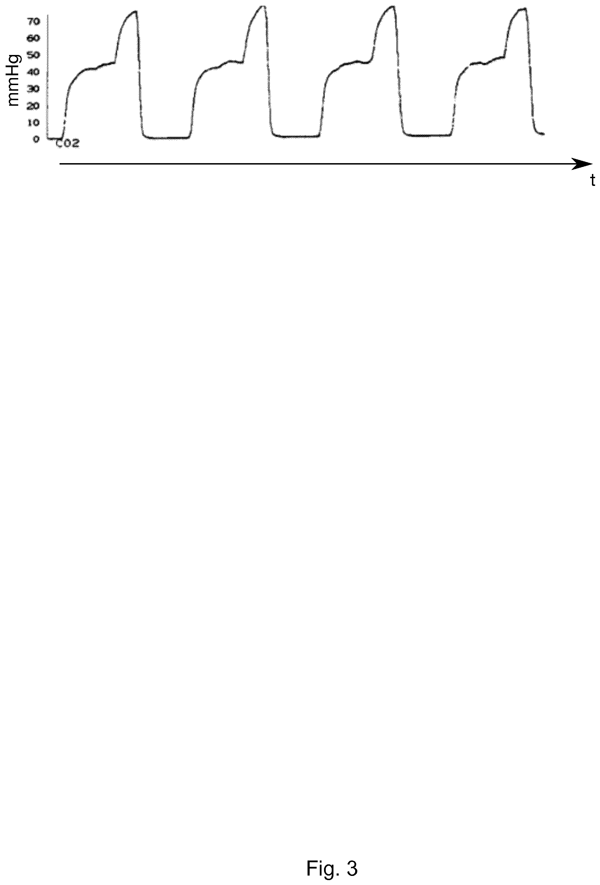

Fig. 3 zeigt ein Kapnogramm (zeitlicher Verlauf einer CO2 Konzentration im mmHg) eines Patienten in einem Ausführungsbeispiel. Das Kapnogramm wurde in einem System mit internem Gasanalysesensor aufgezeichnet. Der Verlauf zeigt einen periodischen Verlauf der innerhalb einer Periode zunächst auf ein Plateau ansteigt. Das Plateau wird gefolgt von einem kurzen Anstieg bevor die Konzentration steil auf Null abfällt. Bei dieser Untersuchung korrelierten die CO2-Konzentrationen im Atemgas gut mit dem arteriellen Kohlendioxidpartialdruck, wobei die höchsten CO2-Konzentrationen mehr als 30mmHg über den Höchstwerten des arteriellen Kohlendioxidpartialdrucks lagen, vgl. auch "End-Tidal CO2 Excretion Waveform and Error with Gas Sampling Line Leak", Zupan, Joanne MD; Martin, Michael MD; Benumof, Jonathan L. MD , Anesthesia & Analgesia: June 1988 - Volume 67 - Issue 6 - ppg 579-581.

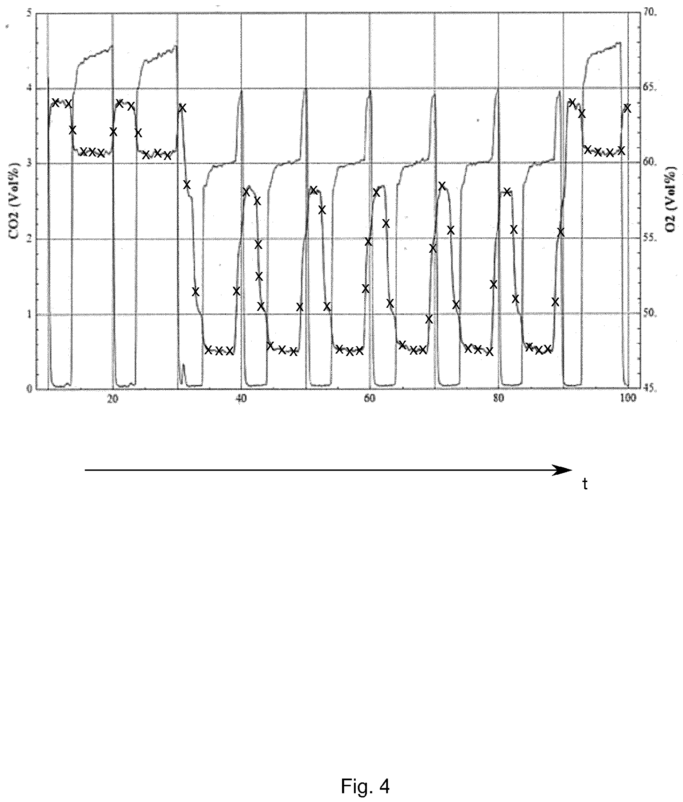

Ähnliche Auffälligkeiten finden sich auch in Kapnogrammen, die im folgenden Ausführungsbeispiel mit einer Simulationslunge (z.B. Michigan-Lunge) im Labor erzeugt wurden. Fig. 4 zeigt Konzentrationsverläufe von Kohlendioxid (ungekennzeichneter Verlauf in Volumenprozent) und Sauerstoff (mit "x" gekennzeichneter Verlauf in Volumenprozent) in einem Ausführungsbeispiel. Dabei wurde ein kleines Leck im Bereich des Probengas-Eingangs/Wasserfalle vgl. Fig. 2, am Patientengas-Modul ab T=30s geöffnet und bei T=90s geschlossen (durchstochener Probengasschlauch). Gleichzeitig mit der CO2-Kurve ist auch die O2-Kurve dargestellt. Neben der in o. g. Veröffentlichung diskutierten Peak-haften Überhöhung des CO2-Verlaufes im Leckage-Fall jeweils zum Ende der Exspirationsphase, findet sich in Folge der Verdünnung durch die Leckage auch gleichzeitig eine Beeinflussung der 02-Kurve statt. Neben einer Änderung der Amplituden- und Mittelwerte der Konzentrationskurven, die jedoch nicht Leckage-spezifisch sind, findet sich aber eine scheinbare Phasenverschiebung zwischen CO2- und O2-Kurve. Kritisch für die Amplitude und Mittelwerte der Konzentrationsverläufe sind z. B. auch Änderungen der Beatmungseinstellungen und Frischgas-Konzentration.

Die im Leckage-Fall beobachtbare Phasenverschiebung zwischen CO2- und O2-Kurve (je nach Konzentrationsverhältnissen und/oder auch zwischen CO2 und Agas- bzw. N2O-Kurve vorhanden) ist absolut messend, d. h. ohne Kenntnis der Konzentrationsverhältnisse der vorangehenden Atemphasen anwendbar. Dies kann ein Vorteil sein, da sich mit diesem Merkmal auch bei wechselnden Konzentrationsverhältnissen und ohne Vorwissen bzw. ohne Messwerte vorangegangener Atemphasen Lecks oder Leckagen zuverlässig detektieren lassen. Als weiteren Vorteil bietet das beschriebene Verfahren die Möglichkeit, Leckagen auch dann zu detektieren, wenn diese zeitlich nicht abrupt auftreten sondern über mehrere Atemphasen kontinuierlich anwachsen. Eine Voraussetzung für dieses Verfahren ist, dass genügend große Beatmungsdruck-Änderungen am Patienten-seitigen Anschlusspunkt der Probengasleitung vorliegen ($\Delta P \geq 10$ hPa). In Ausführungsbeispielen kann als Ähnlichkeitsmaß demnach eine Phasenverschiebung zwischen dem ersten ($CO2$) und dem zweiten Verlauf (z.B. 02) herangezogen werden. Das Bestimmen 16 des Ähnlichkeitsmaßes kann ein Bestimmen einer für ein Leck eigentümlichen Verformung in dem ersten oder dem zweiten Verlauf umfassen, vgl. Fign 3 und 4. Darüber hinaus kann das Ähnlichkeitsmaß ein Maß für eine Symmetrie zwischen dem ersten und dem zweiten Verlauf sein.

In einigen Ausführungsbeispielen kann demnach eine quantitative Auswertung der Konzentrationsverläufe stattfinden. Eine Grundlage können dabei Beatmungsdruck-Schwankungen bilden, die zu Phasenverschiebungen zwischen $CO2$- und 02 bzw. Agas-/N2O-Kurven führen. Diese Phasenverschiebungen werden in den im Folgenden beschriebenen Ausführungsbeispielen mit Hilfe der Kovarianz (Cov) ausgewertet:

$$Cov_{xy} := \frac{2}{n} \sum_{i=1}^{n} \left[ \frac{(x_i - \bar{x})}{Max_x - Min_x} \times \frac{(y_i - \bar{y})}{Max_y - Min_y} \right]$$

mit den maximalen (Max) bzw. minimalen (Min) der Konzentrationswerten innerhalb der betrachteten Atemphase und den Mittelwerten

$$\bar{x} = \frac{1}{n} \sum_{i=1}^{n} x_i,$$

$$\bar{y} = \frac{1}{n} \sum_{i=1}^{n} y_i.$$

Dabei geht der Laufindex i über alle Werte in der betrachteten Atemphase (i = 1... n). Die Werte $x_i$ stehen für die Echtzeit $CO2$-Werte und die $y_i$ für die Echtzeitwerte aus der Gruppe 02, Agas oder N2O. Es gibt in Ausführungsbeispielen also zumindest eine Auswertung pro Atemphase. Im Nicht-Leckage-Fall gilt idealer Weise

$$Cov(CO2, Y) = -1,$$

mit $Y \in \{O_2, AGas, N_2O\}$,
d. h. die normierten und Mittelwerts-befreiten Kurven sind (zumindest theoretisch) komplett antisymmetrisch. Tatsächlich liegen die entsprechenden Covarianz-Werte aber eher zwischen -0,6 und - 0,8. Das Ähnlichkeitsmaß ist demnach im vorliegenden Ausführungsbeispiel eine Kovarianz zwischen dem ersten (CO2) und dem zweiten (02) zeitlichen Verlauf der Konzentrationen. In Ausführungsbeispielen kann daher das Ähnlichkeitsmaß auch ein Maß für die zwischen den Verläufen vorhandene Symmetrie oder Asymmetrie sein. Im Leckage-Fall kommt es zu einer Beatmungsdruckverursachten Phasenverschiebung, die die Covarianz in Richtung positiver Werte verschiebt. Der Schwellwert für die Covarianz zur Erkennung einer Leckage beträgt z.B. -0.4, d. h.
$Cov_{xy}$ > -0.4 => *Leckage!*
Im Allgemeinen ist der Schwellwert abhängig von dem Bauzustand des Patientengasmoduls etc. Daher stellt der Schwellwert keine universelle Größe dar, sondern ist zumindest in manchen Ausführungsbeispielen spezifisch für das verwendete Patientengasmodul. In diesem Ausführungsbeispiel umfasst das Verfahren also ferner ein Vergleichen des Ähnlichkeitsmaßes mit einem Schwellenwert und ein Detektieren des Lecks basierend auf dem Schwellenwertvergleich.
In einigen weiteren Ausführungsbeispielen lässt sich eine verbesserte Unterscheidung zwischen Leckage und Nicht-Leckage z. B. dadurch erreichen, dass zu einem festgelegten Zeitpunkt mit unabhängigen Verfahren die Integrität der Absauge-Strecke festgestellt wird (etwa mit einer Druckprüfung wie weiter unten beschrieben). Dann kann der tatsächliche Kovarianz-Wert ermittelt bzw. der Grenzwert für eine Leckage diesem Messwert angepasst werden (z. B. Grenzwert = 2/3 x Kovarianz-Messwert im Nicht-Leckage-Fall). Insofern kann das Verfahren 10 in einigen Ausführungsbeispielen ferner ein Kalibrieren des Schwellwertes basierend auf einer Referenzmessung an dem Beatmungssystem ohne Leck umfassen.

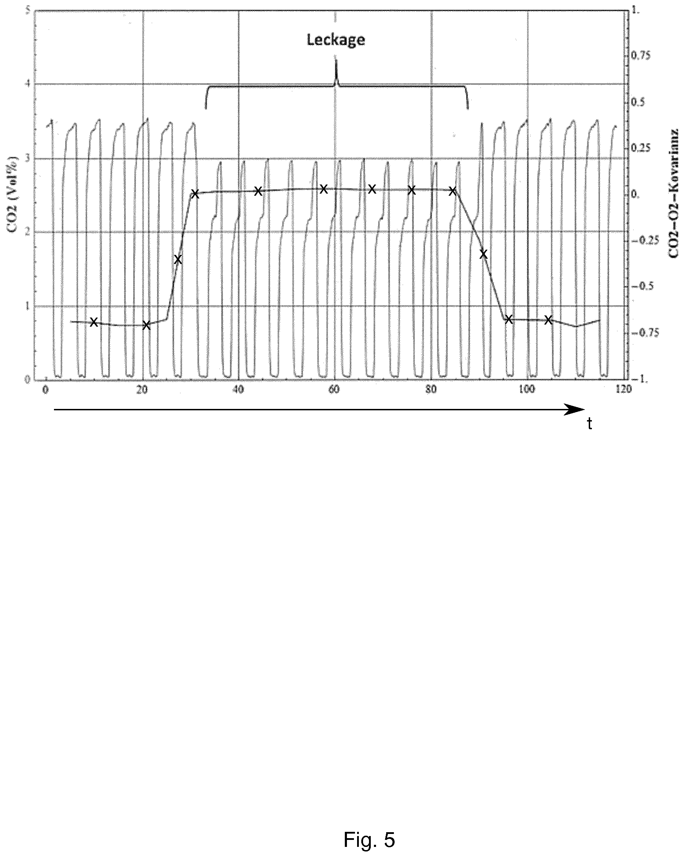

Fig. 5 zeigt einen Kohlendioxid-Konzentrationsverlauf (Verlauf ohne Markierungen) und einen Kohlendioxid-Sauerstoff-Kovarianzverlauf (mit "x" gekennzeichneter Verlauf) in einem Ausführungsbeispiel für die Detektion einer Leckage mit dem oben dargestellten Verfahren und in dem in der Fig. 2 gezeigten System. In der Fig. 5 sind die $CO2$-

Konzentration und die CO2/O2-Kovarianz ohne und mit Leckage (Perforation Absaugeschlauch im Bereich der Wasserfalle) zu sehen. Die Leckage wurde bei t≈30s geöffnet und bei t≈90s wieder geschlossen. Der Verlauf der Kovarianz (gekennzeichnet mit "x") verzeichnet einen deutlichen Sprung, wenn die Leckage geöffnet wird und eignet sich daher, um die Leckage zu detektieren.

In einem weiteren Ausführungsbeispiel werden anstatt von Konzentrationen direkt, Konzentrationsänderungen betrachtet, um das Ähnlichkeitsmaß zu ermitteln. Das Ähnlichkeitsmaß kann dann eine Kovarianz zwischen einem ersten und einem zweiten zeitlichen Verlauf von Konzentrationsänderungen sein. Ähnlich wie im o. g. Fall (Konzentrationskovarianz) beruht die Leckage-Erkennung im vorliegenden Ausführungsbeispiel ebenfalls auf der Kovarianz - allerdings werden in diesem Fall die Änderung (bzw. die Differenzen) der Echtzeitmesswerte berücksichtigt. Vorteil kann hier sein, dass dann auch bei niedrigen Beatmungsfrequenzen signifikante Änderungen deutlicher heraustreten und die unveränderten Teile der Zeitverläufe besser unterdrückt bzw. nicht bewertet werden.

Damit ergibt sich als Delta-Kovarianz:

$$\Delta Cov_{xy} := \frac{2}{n-l} \sum_{i=1+l}^{n} [(\tilde{x}_i - \tilde{x}_{i-l}) \cdot (\tilde{y}_i - \tilde{y}_{i-l})]$$

mit den normalisierten Werten

$$\tilde{x}_i = \frac{1}{\text{Max}_x - \text{Min}_x}\left[x_i - \frac{\text{Max}_x - \text{Min}_x}{2}\right],$$

$$\tilde{y}_i = \frac{1}{\text{Max}_y - \text{Min}_y}\left[y_i - \frac{\text{Max}_y - \text{Min}_y}{2}\right]$$

Als brauchbarer Wert hat sich z.B. l=10 herausgestellt (bei einen Abtastfrequenz von 1/20 ms ergibt sich daraus als Zeitdifferenz 200 ms für die Messwertdifferenzen). Wie im Fall der Kovarianz wird die Summe beispielsweise immer über die aktuelle Atemphase ausgewertet und man erhält dann jeweils einen Wert pro Atemphase. Auch hier kann initial ein Grenzwert zur Unterscheidung zwischen Leckage und Nicht-Leckage ermittelt werden (Kalibrierung). Insofern kann das Verfahren 10 auch in diesem Ausführungsbeispiel ferner ein Vergleichen des Ähnlichkeitsmaßes mit einem Schwellenwert und ein Detektieren des Lecks basierend auf dem Schwellenwertvergleich umfassen. Darüber hinaus kann ein Kalibrieren des Schwellenwertes basierend auf einer Referenzmessung an dem Beatmungssystem ohne Leck stattfinden.

In einem weiteren Ausführungsbeispiel findet in dem Verfahren ein Bestimmen einer Laufzeit des Atemgasgemischs von dem Patienten über eine Probengasleitung 52 zu einem Patientengasmodul 50 statt. Das Verfahren 10 umfasst ferner ein Bestimmen einer Laufzeit von Konzentrationsänderungen in dem Atemgasgemisch und ein Bestimmen des Ähnlichkeitsmaßes basierend auf den Laufzeiten. Bei der absaugenden Patientengasmessung wird ein Gasstrom vom Patienten abgesaugt, vgl. Fig. 2. Die Konzentrationen des Gasgemisches des Gasstroms werden von der Gasmessung ausgewertet. Gewöhnlich besteht das Gasgemisch aus einem Anteil Sauerstoff $\sigma_{O2}$, einem Anteil $CO2 \sigma_{CO2}$, einem Anteil Narkosegas $\sigma_A$ und einem Anteil Lachgas $\sigma_{N2O}$. Hat das Gasgemisch zum Zeitpunkt t die Konzentrationswerte $\sigma_i$, werden diese Werte von der Gasmessung zu einem Zeitpunkt $t + \Delta t_{pat}$ erfasst. Die Ursache für die Verzögerung ist die Laufzeit $\Delta t_{pat}$, die das Gas benötigt, um vom Patienten über die Probengasleitung zum Patientengasmodul zu gelangen. Die Laufzeit kann näherungsweise in der Form

$$\Delta t_{pat} = \frac{L_{sample} \cdot \pi \cdot d^2_{sample}}{4 \cdot \dot{V}_{gas}}$$

bestimmt werden. Der abgesaugte Gasstrom $\dot{V}_{gas}$ benötigt die Zeit $\Delta t_{pat}$, um über die Probengasleitung mit der Länge $L_{sample}$ und dem Querschnitt $\pi \cdot d^2_{sample}$ vom Patienten zum Patientengasmodul zu gelangen.

Der Druck am Patienten wird ebenfalls vom Patientengasmodul erfasst. Durch die zwischen Patienten und Patientengasmodul liegenden Volumina und Strömungswiderstände spiegelt der gemessene Druckwert aber nicht die Verhältnisse am Patienten wieder. Lediglich die Druckdifferenz zwischen den Atemphasen Inspiration und Exspiration kann näherungsweise korrekt wiedergegeben werden. Zudem können Druckschwankungen am Patienten vom Patientengasmodul nahezu zeitgleich erfasst werden, da sich das Drucksignal mit Schallgeschwindigkeit ausbreitet.

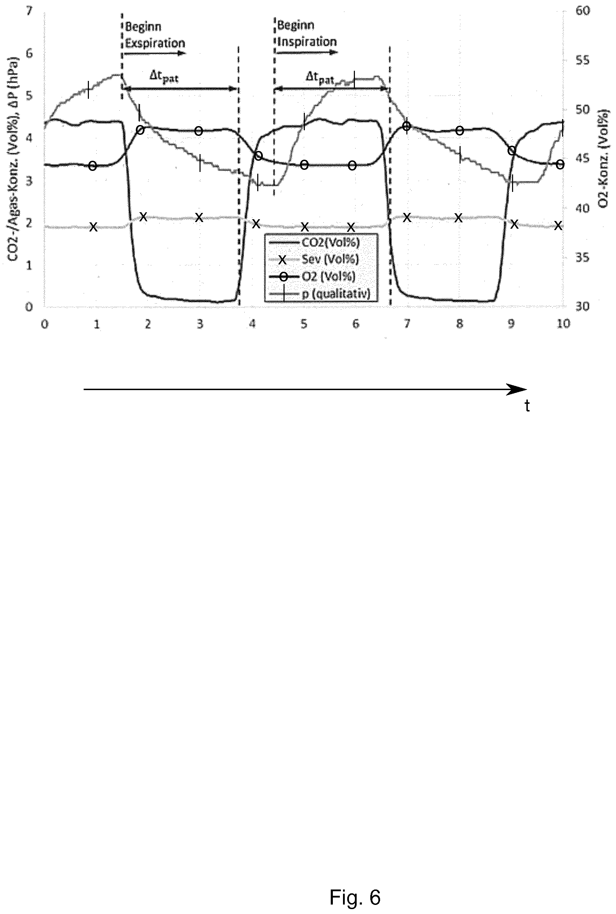

Fig. 6 zeigt Konzentrationsverläufe und Druckverläufe in einem Patientengasmodul in einem Ausführungsbeispiel ohne Leckage. Fig. 6 illustriert gemessene Konzentrationen von $CO_2$ (ohne Markierung), Narkosegas (Sevoflurane mit "x"-Markierung), $O_2$ (mit "o"-Markierung) sowie einen qualitativen Verlauf des Drucks p (mit "|"-Markierung) im Patientengasmodul in Abhängigkeit der Zeit ohne eine Leckage.

Zu Beginn einer Exspirationsphase fällt das Drucksignal im Patientengasmodul ab, da der Atemwegsdruck auf den Exspirationsdruck abfällt. Zu Beginn einer Inspirationsphase steigt das Drucksignal im Patientengasmodul an, da der Atemwegsdruck auf den Inspirationsdruck ansteigt. Diese beiden Zeitpunkte sind in Fig. 6 entsprechend markiert. Zeitverzögert (um $\Delta t_{pat}$) steigt zu Beginn einer Exspirationsphase das $CO_2$-Signal an und die Signale für $O_2$ und Sevoflurane fallen entsprechend ab. Zu Beginn einer Inspirationsphase fällt das $CO_2$-Signal zeitverzögert (um $\Delta t_{pat}$) ab und die Signale für $O_2$ und Sevoflurane steigen entsprechend an. Auch diese Übergänge sind in Fig. 6 markiert. Die Zeitverzögerung $\Delta t_{pat}$ ist gut zu erkennen und hat den Wert $\Delta t_{pat} \approx 2,3s$. Die pneumatische Zeitverzögerung dürfte deutlich niedriger ausfallen, da im Signalverlauf eine Verzögerung der Verarbeitung der Konzentrationssignale ebenfalls enthalten ist.

Deutlich zu erkennen ist, dass im Falle eines Atemphasenwechsels das Drucksignal klar vor den Konzentrationssignalen auf den Wechsel reagiert. Dies und die Tatsache, dass keine weiteren signifikanten Änderungen in den Konzentrationsverläufen auftreten, sprechen für ein System ohne Leckage. Für den Fall, dass keine relevante Leckage die Patientengasmessung beeinflusst, kann jede Änderung der Konzentrationen des Gasgemisches am Patienten, die durch die Atemphasen hervorgerufen werden, frühestens nach der Zeit $\Delta t_{pat}$ detektiert werden.

Für den Fall, dass eine relevante Leckage im Bereich der Patientengasmessung vorhanden ist, verändern sich die strömungsmechanischen Eigenschaften des Systems. Betrachtet man z.B. ein Leck am Ort, wo die Probengasleitung 52 mit dem Patientengasmodul 50 verbunden ist, vgl. Fig. 2, ergibt sich ein Volumenstrom $\dot{V}_{leck}$ der in Abhängigkeit der vorhandenen Druckdifferenz $\Delta p_{leck}$, in das System eindringt und die Konzentrationen des zu messenden Gasgemisches verändert oder aus dem System austritt, die Zusammensetzung des Gasgemisches aber nicht ändert. Für den Fall, dass ein Volumenstrom $\dot{V}_{leck}$ in das System eintritt, setzt sich ein Volumenstrom $\dot{V}_{pump}$, den die Pumpe im Patientengasmodul absaugt, aus einem Anteil $\dot{V}_{sample}$, der durch die Probengasleitung fließt, und $\dot{V}_{leck}$ zusammen. Die beiden Anteile variieren je nach Atemphase. Ist in der Inspirationsphase der Druck am Patienten hoch, ist die Druckdifferenz am Ort der Leckage klein, wohingegen in der Exspirationsphase der Druck am Patienten niedrig und somit die Druckdifferenz am Ort der Leckage groß ist. Diese Druckschwankungen verändern den Volumenstrom $\dot{V}_{leck}$ und somit auch die Konzentrationen der Gase am Ort der Leckage.

Diese Änderungen werden durch das Patientengasmodul 50 mit einer anderen Zeitverzögerung erfasst, als die Änderungen am Patienten. Eine Änderung des Gasgemisches am Ort der Leckage kann vom Patientengasmodul nach der Zeit

$$\Delta t_{leck} = \frac{\Delta L_{sample} \cdot \pi \cdot d^2_{sample}}{4 \cdot \dot{V}_{gas}}$$

erfasst werden. Dabei bezeichnet $\Delta L_{sample}$ hier den Abstand zwischen Leckage-Ort (z. B. am Eingang der Wasserfalle) bis zum Patientengassensor und es gilt: $\Delta L_{sample} < L_{sample}$

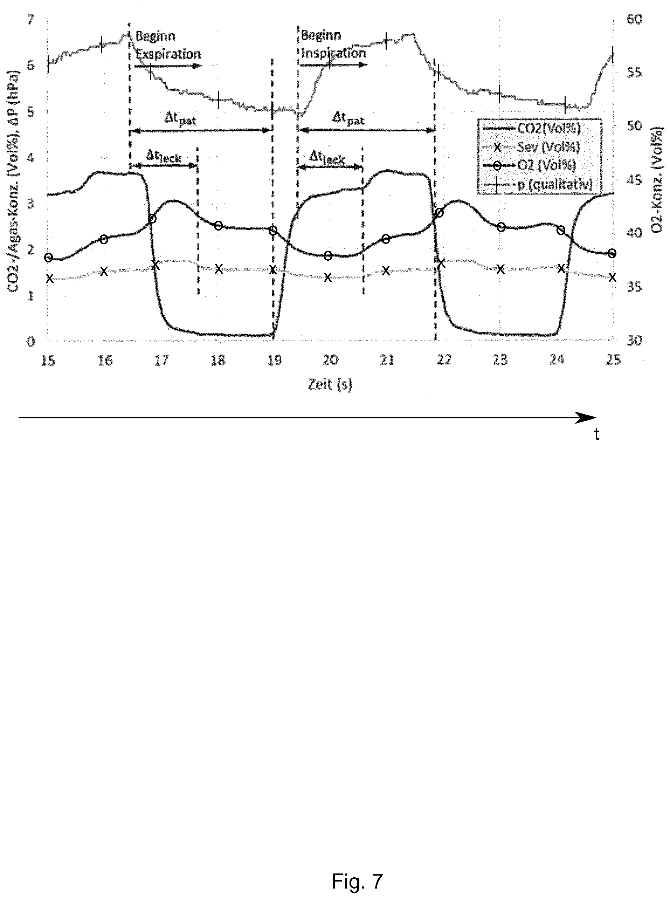

Fig. 7 zeigt Konzentrationsverläufe und Druckverläufe in einem Patientengasmodul in einem Ausführungsbeispiel mit Leckage. Fig. 7 zeigt gemessene Konzentrationen von $CO_2$ (ohne Markierung), Sevoflurane ("x"-Markierung), $O_2$ ("o"-Markierung) und den qualitativen Verlauf des Drucks p ("|"-Markierung) im Patientengasmodul 50 in Abhängigkeit der Zeit mit einer Leckage. Neben den anhand der Fig. 6 erläuterten Verläufen der Konzentrationssignale ergeben sich im Falle einer Leckage weitere Auffälligkeiten. Bezogen auf den Start der Exspirationsphase fallen die Konzentrationen von $O_2$ und Sevoflurane nach einer Zeit $\Delta t_{leck}$ ab. Dies ist eine Folge des erhöhten Differenzdrucks am Ort der Leckage in der Exspirationsphase und der damit verbundenen größeren Verdünnung des abgesaugten Gases durch den vergrößerten, eindringenden Volumenstrom.

Bezogen auf den Start der Inspirationsphase steigt das $CO_2$-Signal nach einer Zeit $\Delta t_{leck}$ an und es fallen gleichzeitig die Konzentrationen von $O_2$ und Sevoflurane ab. Dies ist eine Folge des Beatmungsdruck-bedingten verringerten Differenzdrucks am Ort der Leckage in der Inspirationsphase und der damit verbundenen niedrigeren Verdünnung des abgesaugten Gases durch den verringerten, eindringenden Volumenstrom. In diesem Fall ergibt sich wieder der Wert $\Delta t_{pat} \approx 2,3s$, aber auch der deutlich kleinere Wert $\Delta t_{leck} \approx 1,2s$.

Für den Fall, dass eine relevante Leckage die Patientengasmessung beeinflusst, führt jede Änderung der Konzentrationen des Gasgemisches am Patienten, die mit einer Druckänderung durch einen Wechsel der Atemphasen verbunden ist, zu gemessenen Konzentrationsänderungen nach einer Verzögerung von $\Delta t_{leck}$ und $\Delta t_{pat}$, wobei gilt $\Delta t_{leck}$

$< \Delta t_{pat}.$

Der Indikator für die Feststellung einer Leckage ist das Verhalten der Konzentrationssignale am Patientengasmodul nach dem Beginn einer Atemphase. Veränderungen der Konzentrationen, die erst nach $\Delta t_{pat}$ auftreten, stehen für ein System ohne Leckage, Veränderungen die bereits nach $\Delta t_{leck}$ auftreten, indizieren ein System mit Leckage.

In Ausführungsbeispielen mit dieser Art der Erkennung einer Leckage können ein Indikator für den Beginn der Atemphasen und ein Indikator zur Erfassung der Zeitpunkte der Konzentrationsänderungen am Patientengasmodul 50 bestimmt werden. Indikatoren für den Beginn der Atemphasen sind beispielsweise

- das Drucksignal im Patientengasmodul (siehe Fign. 6 und 7) oder
- ein Signal vom Atemsystem, das den Beginn einer Atemphase anzeigt.

Indikatoren zur Erfassung des Zeitpunkts einer Konzentrationsänderung sind

- eine signifikante Veränderung der ersten Ableitung der Konzentrationswerte in Abhängigkeit der Zeit oder
- eine Bewertung der Konzentrationswerte vor und nach dem Beginn einer Atemphase.

Das oben beschriebene Verfahren 10 ermöglicht eine Detektion einer Leckage in einer absaugenden Patientengasmessung durch die Erfassung der zeitlichen Verzögerung von Konzentrationsänderungen im abgesaugten Gasgemisch nach einer Änderung der Atemphase. Ausführungsbeispiele können demnach ein Bestimmen der Laufzeit basierend auf Konzentrationsänderungen über der Zeit oder basierend auf einer Bewertung von Konzentrationsänderungen vor und nach einem Beginn von Atemphasen umfassen. Das Ähnlichkeitsmaß kann demnach durch eine zeitliche Verzögerung bestimmt sein oder einer zeitlichen Verzögerung entsprechen.

In einem weiteren Ausführungsbeispiel umfasst das Verfahren 10 ferner ein Durchführen einer Druckmessung an dem Beatmungssystem, wenn ein Leck detektiert wurde. Ist nun durch ein o. g.

Verfahren eine Leckage-Indikation gegeben, so erfolgt zumindest in manchen Ausführungsbeispielen nun ein Druckmess-Manöver, das dann eine valide Aussage zum Leckage-Status ermöglicht. Der Ablauf des Manövers, das zu einem beliebigen Zeitpunkt während einer Beatmung/Operation erfolgen kann und die Variation des Messdruckes in dem Gasmonitor (bzw. in der Messzelle des Gasmonitors) auf Grund der aufgeprägten Beatmungsdruckschwankungen, untergliedert sich gemäß:

1. Ausschalten der Probengaspumpe,
2. Ausgangseitiges Dichtsetzen des Patientengasmoduls (mittels eines entsprechenden elektromagnetischen Ventils),
3. Ermittlung des Inspirations- und Exspirationsdrucks im Patientengasmodul,
4. Berechnung der Druckdifferenz zwischen Exspirations- und Inspirationsdruckwert im Patientengasmodul, und
5. Vergleich mit den Druckwerten im Beatmungskreis bzw. mit den Einstellwerten am Beatmungs-Ventilator: Bei einem Unterschied > 3 hPa erfolgt eine Leckage-Alarmierung.

Durch diesen Ablauf kann in manchen Ausführungsbeispielen sichergestellt werden, dass die Häufigkeit dieses Manövers, das ja mit dem Ausfall der Gasmessfunktion verbunden ist so klein wie möglich ausfällt und nur dann ausgeführt wird, wenn ein begründeter Leckage-Verdacht besteht. Gleichzeitig kann eine Alarmierung auch nur dann ausgelöst werden, wenn eine eindeutige Leckage vorliegt.

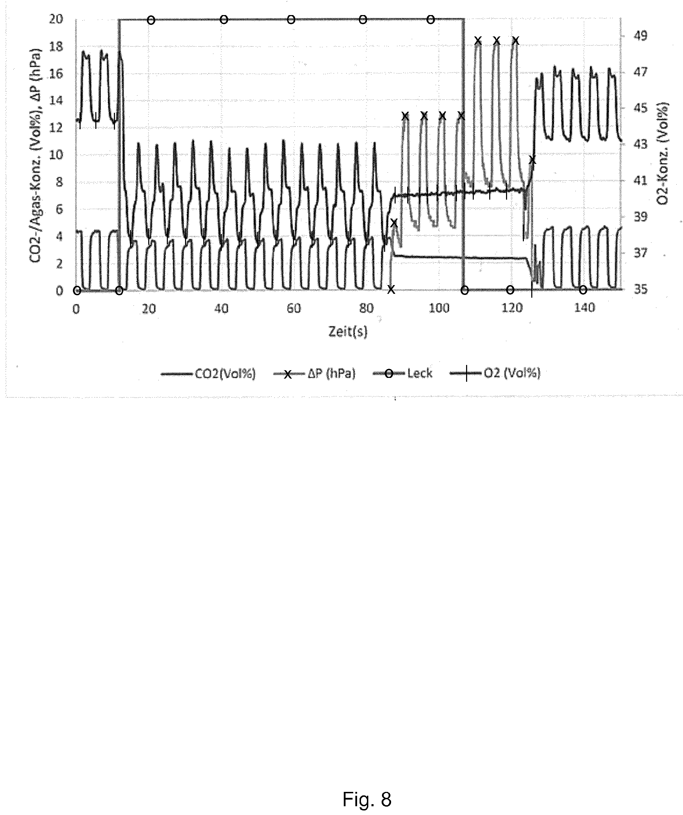

Fig. 8 zeigt Konzentrationsverläufe und Druckverläufe in einem Patientengasmodul 50 in einem weiteren Ausführungsbeispiel mit Leckage. Fig. 8 zeigt Verläufe für CO2 (ohne Markierung), 02 ("|"-Markierung), den Leckageabschnitt ("o"-Markierung) und den Druckverlauf ΔP (Druck im Patientengasmodul relativ zum Umgebungsdruck, "x" Markierung) mit und ohne Leckage. Das Druckmanöver mit ausgeschalteter Probengaspumpe ist gekennzeichnet durch konstante 02- und CO2-Verläufe. Zur besseren Darstellung der Signifikanz dieser Prüfung wurde das Druckmanöver mit (Zeitbereich t ≈ 90s ... 100s) und ohne Leckage (Zeitbereich t ≈ 105s ... 125s) durchgeführt. Die Unterschiede sind in der Fig. 8 deutlich zu erkennen.

Die in der vorstehenden Beschreibung, den Ansprüchen und den Zeichnungen offenbarten Merkmale können sowohl einzeln als auch in beliebiger Kombination für die Verwirklichung von Ausführungsbeispielen in ihren verschiedenen Ausgestaltungen von Bedeutung sein und - soweit sich nicht aus der Beschreibung etwas anderes ergibt - beliebig miteinander kombiniert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß eines Ausführungsbeispiels kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch Schreiben in eine oder mehrere Speicherstellen eine Aktion bewirken, veranlassen oder durchführen sowie andere Geräte, Maschinen und Komponenten ansteuern.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**Patentansprüche**

1. Verfahren (10) zur Detektion eines Lecks in einem Patientengasmodul, das einen kontinuierlichen Probengasstrom von einem beatmeten Patienten (20) absaugt und analysiert, mit

   Bestimmen (12) eines ersten zeitlichen Verlaufs einer Kohlendioxidkonzentration in einem Atemgasgemisch des Patienten (20);

   Bestimmen (14) eines zweiten zeitlichen Verlaufs einer Konzentration eines anderen, sich von Kohlendioxid unterscheidenden Gases in dem Atemgasgemisch;

Bestimmen (16) eines statistischen Ähnlichkeitsmaßes zwischen dem ersten und dem zweiten zeitlichen Verlauf, wobei das Ähnlichkeitsmaß einer Phasenverschiebung zwischen dem ersten und dem zweiten Verlauf entspricht; und
Detektieren (18) des Lecks basierend auf dem Ähnlichkeitsmaß.

2. Verfahren (10) gemäß Anspruch 1, wobei das andere Gas Sauerstoff, Lachgas oder ein Narkosegas ist.

3. Verfahren (10) gemäß einem der vorangehenden Ansprüche, wobei das Bestimmen (16) des Ähnlichkeitsmaßes ein Bestimmen einer für ein Leck eigentümlichen Verformung in dem ersten oder dem zweiten Verlauf umfasst.

4. Verfahren (10) gemäß einem der vorangehenden Ansprüche, wobei das Ähnlichkeitsmaß ein Maß für eine Symmetrie zwischen dem ersten und dem zweiten Verlauf ist.

5. Verfahren (10) gemäß einem der vorangehenden Ansprüche, wobei das Ähnlichkeitsmaß eine Kovarianz zwischen dem ersten und dem zweiten zeitlichen Verlauf der Konzentrationen ist.

6. Verfahren (10) gemäß einem der vorangehenden Ansprüche, wobei das Ähnlichkeitsmaß eine Kovarianz zwischen einem ersten und einem zweiten zeitlichen Verlauf von Konzentrationsänderungen ist.

7. Verfahren (10) gemäß einem der vorangehenden Ansprüche, ferner umfassend Vergleichen des Ähnlichkeitsmaßes mit einem Schwellenwert und Detektieren des Lecks basierend auf dem Schwellenwertvergleich.

8. Verfahren (10) gemäß Anspruch 7, ferner umfassend Kalibrieren des Schwellenwertes basierend auf einer Referenzmessung an dem Beatmungssystem ohne Leck.

9. Verfahren (10) gemäß einem der vorangehenden Ansprüche, ferner umfassend Bestimmen einer Laufzeit des Atemgasgemischs von dem Patienten (20) über eine Probengasleitung (52) zu einem Patientengasmodul (50).

10. Verfahren (10) gemäß Anspruch 9, ferner umfassend Bestimmen einer Laufzeit von Konzentrationsänderungen in dem Atemgasgemisch und Bestimmen des Ähnlichkeitsmaßes basierend auf den Laufzeiten.

11. Verfahren (10) gemäß Anspruch 10, ferner umfassend Bestimmen der Laufzeit basierend auf Konzentrationsänderungen über der Zeit oder basierend auf einer Bewertung von Konzentrationsänderungen vor und nach einem Beginn von Atemphasen.

12. Verfahren (10) gemäß einem der vorangehenden Ansprüche, ferner umfassend Durchführen einer Druckmessung an dem Beatmungssystem (40), wenn ein Leck detektiert wurde.

13. Computerprogramm mit einem Programmcode zur Durchführung eines der Verfahren gemäß einem der vorangehenden Ansprüche, wenn der Programmcode auf einem Computer, einem Prozessor oder einer programmierbaren Hardwarekomponente ausgeführt wird.

14. Vorrichtung (30) zum Durchführen eines der Verfahren (10) eines der Ansprüche 1 bis 12.

15. Beatmungssystem (40) für einen Patienten (20) mit der Vorrichtung (30) gemäß Anspruch 14.

**Claims**

1. Method (10) for detecting a leak in a patient gas module which suctions and analyses a continuous stream of sample gas from a ventilated patient (20), the method comprising
determining (12) a first time curve of a carbon dioxide concentration in a respiratory gas mixture of the patient (20);
determining (14) a second time curve of a concentration of another gas in the respiratory gas mixture, which other gas is different from carbon dioxide;
determining (16) a statistical degree of similarity between the first and the second time curve, wherein the degree of similarity corresponds to a phase shift between the first and the second time curve; and detecting (18) the leak on the basis of the degree of similarity.

**2.** Method (10) according to Claim 1, wherein the other gas is oxygen, nitrous oxide or an anaesthetic gas.

**3.** Method (10) according to one of the preceding claims, wherein determining (16) the degree of similarity comprises determining a deformation in the first or the second curve, which deformation is typical of a leak.

**4.** Method (10) according to one of the preceding claims, wherein the degree of similarity is a measure of a symmetry between the first and the second curve.

**5.** Method (10) according to one of the preceding claims, wherein the degree of similarity is a covariance between the first and the second time curve of the concentrations.

**6.** Method (10) according to one of the preceding claims, wherein the degree of similarity is a covariance between a first and a second time curve of concentration changes.

**7.** Method (10) according to one of the preceding claims, further comprising comparing the degree of similarity with a threshold value, and detecting the leak on the basis of the threshold value comparison.

**8.** Method (10) according to Claim 7, further comprising calibrating the threshold value on the basis of a reference measurement at the ventilation system without a leak.

**9.** Method (10) according to one of the preceding claims, further comprising determining a running time of the respiratory gas mixture from the patient (20) to a patient gas module (50) via a sample gas line (52).

**10.** Method (10) according to Claim 9, further comprising determining a running time of concentration changes in the respiratory gas mixture, and determining the degree of similarity on the basis of the running times.

**11.** Method (10) according Claim 10, further comprising determining the running time on the basis of concentration changes over time or on the basis of an evaluation of concentration changes before and after a start of breathing phases.

**12.** Method (10) according to one of the preceding claims, further comprising carrying out a pressure measurement at the ventilation system (40) when a leak has been detected.

**13.** Computer program with a program code for carrying out one of the methods according to one of the preceding claims, when the program code is executed on a computer, a processor or a programmable hardware component.

**14.** Device (30) for carrying out one of the methods (10) according to one of Claims 1 to 12.

**15.** Ventilation system (40) for a patient (20) with the device (30) according to Claim 14.


**Revendications**

**1.** Procédé (10) de détection d'une fuite dans un module de gaz de patient, qui aspire et analyse un flux de gaz échantillon continu chez un patient ventilé (20), le procédé comprenant les étapes suivantes déterminer (12) une première variation dans le temps d'une concentration de dioxyde de carbone dans un mélange de gaz respiratoire du patient (20) ; déterminer (14) une deuxième variation dans le temps d'une concentration d'un autre gaz, différant du dioxyde de carbone, dans le mélange de gaz respiratoire ;
déterminer (16) un niveau de similitude statistique entre la première et la deuxième variation dans le temps, le niveau de similitude correspondant à un déphasage entre la première et la deuxième variation ; et
détecter (18) la fuite sur la base de la mesure de similitude.

**2.** Procédé (10) selon la revendication 1, l'autre gaz étant de l'oxygène, du protoxyde d'azote ou un gaz anesthésique.

**3.** Procédé (10) selon l'une des revendications précédentes, la détermination (16) du niveau de similitude comprenant la détermination d'une déformation propre à une fuite dans la première ou la deuxième variation.

**4.** Procédé (10) selon l'une des revendications précédentes, le niveau de similitude étant un niveau de symétrie entre la première et la deuxième variation.

5. Procédé (10) selon l'une des revendications précédentes, le niveau de similitude étant une covariance entre la première et la deuxième variation dans le temps des concentrations.

6. Procédé (10) selon l'une des revendications précédentes, le niveau de similarité étant une covariance entre une première et une deuxième variation dans le temps de changements de concentration.

7. Procédé (10) selon l'une des revendications précédentes, comprenant en outre la comparaison du niveau de similitude à une valeur de seuil et la détection de la fuite sur la base de la comparaison à une valeur de seuil.

8. Procédé (10) selon la revendication 7, comprenant en outre l'étalonnage de la valeur de seuil sur la base d'une mesure de référence au niveau du système de ventilation sans fuite.

9. Procédé (10) selon l'une des revendications précédentes, comprenant en outre la détermination d'un temps de propagation du mélange de gaz respiratoire du patient (20) à un module de gaz de patient (50) par le biais d'une conduite de gaz échantillon (52).

10. Procédé (10) selon la revendication 9, comprenant en outre la détermination d'un temps de propagation de changements de concentration dans le mélange de gaz respiratoire et la détermination du niveau de similitude sur la base des temps de fonctionnement.

11. Procédé (10) selon la revendication 10, comprenant en outre la détermination du temps de propagation sur la base de changements de concentration au cours du temps ou sur la base d'une évaluation de changements de concentration avant et après un début de phases de respiration.

12. Procédé (10) selon l'une des revendications précédentes, comprenant en outre la réalisation d'une mesure de pression au niveau du système de ventilation (40) lorsqu'une fuite a été détectée.

13. Logiciel comprenant un code de programme destiné à exécuter l'un des procédés selon l'une des revendications précédentes, lorsque le code de programme est exécuté sur un ordinateur, un processeur ou un composant matériel programmable.

14. Dispositif (30) destiné à mettre en œuvre l'un des procédés (10) selon l'une des revendications 1 à 12.

15. Système de ventilation (40) destiné à un patient (20), ledit système comprenant le dispositif (30) selon la revendication 14.

| Bestimmen eines ersten zeitlichen Verlaufs einer Kohlendioxidkonzentration in einem Atemgasgemisch des Patienten |

12

| Bestimmen eines zweiten zeitlichen Verlaufs einer Konzentration eines anderen Gases in dem Atemgasgemisch |

14

| Bestimmen eines statistischen Ähnlichkeitsmaßes zwischen dem ersten und dem zweiten zeitlichen Verlauf |

16

| Detektieren des Lecks basierend auf dem Ähnlichkeitsmaß |

18

10

Fig. 1

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8033280 B2
- WO 2014068000 A1
- WO 2004076944 A2

**In der Beschreibung aufgeführte Nicht-Patentliteratur**

- **ZUPAN, JOANNE MD ; MARTIN, MICHAEL MD ; BENUMOF, JONATHAN L. MD.** End-Tidal CO2 Excretion Waveform and Error with Gas Sampling Line Leak. *Anesthesia & Analgesia,* Juni 1988, vol. 67 (6), 579-581